(19) Europäisches Patentamt
European Patent Office
Office européen des brevets

(11) **EP 1 412 079 B1**

(12) **EUROPÄISCHE PATENTSCHRIFT**

(45) Veröffentlichungstag und Bekanntmachung des Hinweises auf die Patenterteilung:
**03.01.2007 Patentblatt 2007/01**

(21) Anmeldenummer: **02754874.2**

(22) Anmeldetag: **11.07.2002**

(51) Int Cl.:
**B01J 19/00** (2006.01)

(86) Internationale Anmeldenummer:
**PCT/EP2002/007761**

(87) Internationale Veröffentlichungsnummer:
**WO 2003/006152 (23.01.2003 Gazette 2003/04)**

(54) **VERFAHREN UND VORRICHTUNG ZUM AUFFINDEN VON STOFFGEMISCHEN FÜR SUBSTANZBIBLIOTHEKEN**

METHOD AND DEVICE FOR FINDING SUBSTANCE MIXTURES FOR SUBSTANCE LIBRARIES

PROCEDE ET DISPOSITIF DE DETECTION DE MELANGES DE MATIERES CONNUES POUR DES BIBLIOTHEQUES DE SUBSTANCES

(84) Benannte Vertragsstaaten:
**AT BE BG CH CY CZ DE DK EE ES FI FR GB GR IE IT LI LU MC NL PT SE SK TR**

(30) Priorität: **11.07.2001 DE 10133097**

(43) Veröffentlichungstag der Anmeldung:
**28.04.2004 Patentblatt 2004/18**

(73) Patentinhaber: **Analyticon Discovery GmbH 14473 Potsdam (DE)**

(72) Erfinder:
• **JAKUPOVIC, Jasmin**
 **10707 Berlin (DE)**
• **BINKELE, Heidrun**
 **14548 Ferch-Mittelbusch (DE)**
• **WOLF, Dietmar**
 **10557 Berlin (DE)**
• **SIEMS, Karsten**
 **14557 Langerwisch (DE)**

(74) Vertreter: **Weisse, Renate et al Patentanwälte Weisse & Wolgast Bleibtreustrasse 38 10623 Berlin (DE)**

(56) Entgegenhaltungen:
• LAMBERT P-H ET AL: "QUALITY ASSESSMENT OF COMBINATORIAL POOLED LIBRARIES USING MASS SPECTROMETRY" COMBINATORIAL CHEMISTRY AND HIGH THROUGHPUT SCREENING, HILVERSUM, NL, Bd. 4, Nr. 4, Juni 2001 (2001-06), Seiten 317-332, XP008017386 ISSN: 1386-2073
• HOSTETTMANN K ET AL: "SEARCH FOR NEW LEAD COMPOUNDS FROM HIGHER PLANTS" CHIMIA, AARAU, CH, Bd. 54, Nr. 11, 2000, Seiten 652-657, XP008017376 ISSN: 0009-4293
• JIANG Y ET AL: "ON-LINE COUPLING OF HOLLOW FIBER MEMBRANES WITH ELECTROSPRAY IONIZATION MASS SPECTROMETRY FOR CONTINUOUS AFFINITY SELECTION, CONCENTRATION AND IDENTIFICATION OF SMALL-MOLECULE LIBRARIES" JOURNAL OF MASS SPECTROMETRY, WILEY, CHICHESTER, GB, Bd. 36, Nr. 6, 2001, Seiten 664-669, XP008017384 ISSN: 1076-5174
• NDJOKO K ET AL: "DETERMINATION OF PYRROLIZIDINE ALKALOIDS IN SENECIO SPECIES BY LIQUID CHROMATOGRAPHY/ THERMOSPRAY-MASS SPECTROMETRY AND LIQUID CHROMATOGRAPHY/NUCLEAR MAGNETIC RESONANCE SPECTROSCOPY" PLANTA MEDICA, THIEME, STUTTGART, DE, Bd. 65, Nr. 6, 1999, Seiten 562-566, XP008017380 ISSN: 0032-0943
• WOLFENDER J-L ET AL: "THE IMPORTANCE OF LC-MS AND LC-NMR IN THE DISCOVERY OF NEW LEAD COMPOUNDS FROM PLANTS" PHARMACEUTICAL BIOLOGY, SWETS AND ZEITLINGER, LISSE,, NL, Bd. 38, Nr. SUPPL, 2000, Seiten 41-54, XP008017373 ISSN: 1388-0209

EP 1 412 079 B1

- FANG L ET AL: "EVALUATION OF EVAPORATIVE LIGHT-SCATTERING DETECTOR FOR COMBINATORIAL LIBRARY QUANTITATION BY REVERSED PHASE HPLC" JOURNAL OF COMBINATORIAL CHEMISTRY, AMERICAN CHEMICAL SOCIETY, WASHINGTON, US, Bd. 2, Nr. 3, Mai 2000 (2000-05), Seiten 254-257, XP001157527 ISSN: 1520-4766

- HSU B H ET AL: "APPLICATION OF EVAPORATIVE LIGHT SCATTERING DETECTION TO THE CHARACTERIZATION OF COMBINATORIAL AND PARALLEL SYNTHESIS LIBRARIES FOR PHARMACEUTICAL DRUG DISCOVERY" JOURNAL OF CHROMATOGRAPHY. BIOMEDICAL APPLICATIONS, ELSEVIER, AMSTERDAM, NL, Bd. 725, Nr. 1, 2. April 1999 (1999-04-02), Seiten 103-112, XP001157525 ISSN: 0378-4347

## Beschreibung

## Technisches Gebiet

Die Erfindung betrifft ein Verfahren zum Auffinden von Stoffgemischen, die zum Aufbau von Substanzbibliotheken geeignet sind, welche niedermolekulare, organische Substanzen enthalten, mit den Schritten

(a) Aufbereitung der Gemische für Analysezwecke

(b) Trennung der Gemische mittels chromatographischer oder elektrophoretischer Methoden, und

(c) Identifizierung von niedermolekularen, organischen Substanzen in dem jeweiligen Gemisch durch Erstellung eines Profils aus wenigstens zwei physikalischen Parametern für jedes Gemisch durch Detektion jeder Fraktion mit wenigstens zwei geeigneten Detektoren zur Charakterisierung der in dem Gemisch enthaltenen Substanzen, Vergleich der so erhaltenen Parameter mit den Parametern einer Parametersammlung bekannter Substanzen und Ermittlung der in dieser Sammlung bekannten Substanzen für jedes Gemisch.

Die Erfindung betrifft weiterhin eine Vorrichtung zur Durchführung dieses Verfahrens, sowie die Verwendung des Verfahrens.

## Stand der Technik

Substanzbibliotheken sind u.a. für pharmazeutische Zwecke von kommerziellen und wissenschaftlichen Interesse. Die Bewertung der Eignung von Gemischen, mit denen solche Substanzbibliotheken aufgebaut werden, erfolgt üblicherweise nach der Anzahl der darin enthaltenen geeigneten Substanzen und der Menge in welcher diese Substanzen vorliegen.

Insbesondere Naturstoffe bilden eine außergewöhnlich interessante Quelle für neue Moleküle, die für therapeutische Zwecke getestet werden. Diese Tests sind teuer, langwierig und nur eine geringe Zahl der Moleküle ist am Ende tatsächlich biologisch aktiv. Es ist dementsprechend wichtig, eine Vielzahl von neuen Molekülen in einer ausreichenden Menge für die Tests zur Verfügung zu stellen. Weiterhin sollte die Molekülstruktur bekannt sein, um eine teure Wiederholung von Tests an bereits bekannten und getesteten Molekülen zu vermeiden.

Üblicherweise wird die Molekülstruktur von Naturstoff-Molekülen oder anderen isolierten Reinsubstanzen durch Interpretation der Massenspektren und ein- und zweidimensionaler NMR-Spektren aufgeklärt. Hierfür ist erhebliche Erfahrung und Sachkenntnis erforderlich. Die Isolierung und Strukturaufklärung braucht erhebliche Zeit und ist daher für massenweises Screening von Naturstoffen nur bei erheblichem Zeit- und Personalaufwand zu verwirklichen.

Zur effektiven Isolierung von niedermolekularen Substanzen ist es erforderlich, geeignete Substanzgemische, zum Beispiel geeignete Biomaterialien auszuwählen. Es sollen nur solche Gemische in den zeit- und arbeitsintensiven Isolierungsprozess eingeschleust werden, die eine Mindestzahl an Substanzen in ausreichender Menge enthalten. Diese Substanzen sollten vorher noch nicht isoliert worden sein und nicht zur pharmakologischen Testung zur Verfügung gestanden haben.

Wesentliche Eigenschaften von Stoffen, die für den Aufbau von Substanzbibliotheken geeignet sind, sind also die Neuheit und das Vorliegen in ausreichender Menge. Die Stoffe sollten zudem eindeutig identifizierbar sein. Die Identifizierung kann zum Beispiel über die Struktur erfolgen.

Die Erkennung von bereits isolierten Verbindungen erfolgt im allgemeinen durch Vergleich chromatographischer, spektroskopischer und in einigen Fällen chemischer Parameter. Es ist bekannt, dass je nach Aussagekraft und Spezifität der ermittelten Parameter eine Kombination aus mehreren Parametern zur sicheren Identifizierung der Substanzen eingesetzt werden muß.

Zur Identifizierung von flüchtigen niedermolekularen Naturstoffen ist es bekannt, die Trennung mittels Gaschromatographie vorzunehmen und als Detektor ein Massenspektrometer zu verwenden. Dabei werden neben absoluten Retentionszeiten auch relative Retentionszeiten (Retentionsindizes) als Parameter verwendet, die auf Standardsubstanzen bezogen werden. Dieses Verfahren ist nur für flüchtige Verbindungen und damit nur für einen kleinen Teil der Stoffe geeignet.

Aus der Veröffentlichung von K.Hostettmann, J.-L. Wolfender und S.Rodriguez in Planta Medica **63** (1997) 2-10 mit dem Titel "Rapid Detection and Subsequent Isolation of Bioactive Constituents of Crude Plant Extracts" ist ein Verfahren bekannt, bei welchem eine Hochleistungs-Flüssigchromatographie (HPLC)-Säule mit einem Ultraviolett-Photodiodendetektor (UV-DAD), einem NMR-Spektrometer oder einem Massenspektrometer gekoppelt wurde. Die alleinige Kopplung der HPLC mit der UV-Spektroskopie erlaubt nur die Zuordnung der Peaks zu Stoffklassen, so daß Substanzen, die der gleichen Stoffklasse angehören, nur über Unterschiede in den Retentionszeiten identifizierbar sind. Dieser Nachteil tritt bei der Kopplung mit einem NMR-Spektrometer nicht auf. Die Auswertung von NMR-Spektren ist jedoch nicht automatisierbar. Dies ist bei dem genannten Stand der Technik auch bei der Kopplung der HPLC mit einem Massenspektrometer nicht möglich. Während Massenspektren, die bei der Kopplung mit der Gaschromatographie erhalten werden, unter standardisierten Bedingungen hinsichtlich des Auftretens bestimmter Massen und Intensitäten sehr reproduzierbar und daher einfach in Datenbanken zu speichern und zu suchen sind, treten bei den bei der LC/MS eingesetzten Ionisierungverfahren bestimmte Fragmente und Addukte unregelmäßig auf, die eine Datenbanksuche, die auf einem einfachen Mustervergleich beruht, nicht möglich macht.

Aus einem Vortrag von Holm Sommer und Erwin Keil der Firma PE SCIEX, "8 Jahre Einsatz der LCMS-Technik von PE SCIEX in Forschung und Routine" ist ein Verfahren bekannt, das es gestattet, während der Aufnahme von LC/MS-Spektren die Polarität des Elektrosprays in sehr kurzen Abständen zu wechseln. Durch Auswertung der erhaltenen Addukte in den positiven und negativen Spektren ist es möglich, Molmassen eindeutig zu bestimmen.

Aus der Veröffentlichung von C.E. Kibbey in Molecular Diversity 1 (1995) 247-258 mit dem Titel "Quantitation of combinatorial libraries of small organic molecules by normalphase HPLC with evaporative light-scattering detection" ist ein Verfahren bekannt, bei dem die Quantifizierung von Substanzen in einer HPLC-Fraktion mittels eines Lichtstreu-Detektors (ELSD) erfolgt. Dieser Detektor hat den Vorteil, daß das Signal, im Gegensatz zu zum Beispiel UV-Detektoren, im wesentlichen von der Molekülstruktur unabhängig ist und daher eine gute Quantifizierung erlaubt. Der Detektor kann mit der Normalphasen-HPLC zur Quantifizierung strukturell ähnlicher Substanzen verwendet werden, die sich im Chromophor unterscheiden. Der Detektor kann insbesondere bei der Qualitätskontrolle von Substanzbibliotheken verwendet werden, die mittels kombinatorischer Chemie erstellt wurden.

**Offenbarung der Erfindung**

Der Erfindung liegt die Aufgabe zugrunde, ein Verfahren nach dem Oberbegriff des Anspruchs 1 zu schaffen, mit welchem Gemische, die zum Aufbau von Substanzbibliotheken geeignet sind, schneller und kostengünstiger gefunden und identifiziert werden können.

Erfindungsgemäß wird die Aufgabe dadurch gelöst, daß

(d) eine Quantifizierung der so identifizierten neuen und bekannten Substanzen vorgenommen wird,

(e) die Gemische, bei welchen ausgewählte Substanzen in einer Mindestzahl und/oder Mindestmenge vorliegen, ausgewählt werden,

(f) der erste physikalische Parameter die Retentionszeit ist und

(g) der zweite physikalische Parameter die Molmasse ist, die mittels Massenspektrometrie ermittelt wird.

Das Parameterprofil umfasst die Werte für die Retentionszeit und die Molmasse. Die Erfassung dieses Parameterprofils erlaubt für viele Substanzen bereits vor der präparativen Trennung sowohl die Quantifizierung als auch die Identifizierung einer Substanz in einem Gemisch.

Bei dem Verfahren werden alle Gemische und alle isolierten Reinsubstanzen vorzugsweise unter standardisierten Bedingungen chromatographisch vermessen. Die physikalischen Parameter dienen einerseits zur Quantifizierung der in einem Gemisch vorhandenen Substanzen. Dadurch kann die präparative Trennung von Gemischen vermieden werden, die nicht ausreichend erfolgversprechend sind.

Andererseits dienen die aufgenommenen Werte für jeden Parameter jedes Gemisches bzw. jeder Reinsubstanz der strukturellen Charakterisierung und können jeweils unabhängig voneinander mit einer geeigneten Datenbank verglichen werden. Auf diese Weise ist es möglich, in Gemischen, die noch nicht zur Isolierung von Reinsubstanzen im präparativen Maßstab aufgetrennt wurden, die Verbindungen, die bereits bei der Trennung anderer Gemische als Reinsubstanzen isoliert werden konnten und die schon als Reinsubstanzen vermessen wurden, zu identifizieren. Durch Vergleich der Werte der physikalischen Parameter der Gemische untereinander ist es möglich eine Priorisierung der Extrakte für die folgende präparative Auftrennung zu erstellen, so daß die Isolierung redundanter Verbindungen weitgehend vermieden werden kann. Im Vergleich mit anderen Auswahlkriterien müssen bei diesem Verfahren erheblich weniger Extrake präparativ aufgetrennt werden, was zu Kosteneinsparungen führt und das Auffinden von bisher pharmakologisch nicht oder nicht ausreichend getesteten Stoffen effektiver gestaltet.

Je nach Anwendungszweck der Substanzbibliothek kann dabei die erforderliche Mindestzahl an ausgewählten Substanzen und die Mindestmenge, in der diese Substanzen vorliegen sollen variieren. Ebenso kann sich das Kriterium, nach den Substanzen ausgewählt werden, ändern. Für einige Bibliotheken ist es erforderlich, daß die Substanz neu ist, während eine Auswahl einer Substanz in anderen Fällen schon dann erfolgt, wenn eine Mindestmenge vorliegt.

Aus den ausgewählten, hinreichend erfolgversprechenden Gemischen werden vorzugsweise Reinsubstanzen isoliert und einer Substanzbibliothek hinzugefügt. Auf diese Weise kann mit geeigneten Substanzen eine Bibliothek aufgebaut werden, welche die Basis für das Auffinden weiterer Substanzen bildet. Dies erfolgt derart, daß die Substanzbibliothek nur solche Substanzen umfasst, deren Parameter in der Parametersammlung vorhanden sind oder hinzugefügt werden. Dann werden der Substanzbibliothek nur neue Substanzen hinzugefügt und bekannte Substanzen als solche erkannt.

Vorzugsweise sind die physikalischen Parameter, die vor der Trennung ermittelt werden, die gleichen, die nach der Trennung an der Reinsubstanz gemessen werden. Dies erlaubt die Verifizierung und Verbesserung der Richtigkeit der Parameter an der Reinsubstanz. Die Parameter, die nicht am nicht-getrennten Gemisch gemessen werden, können der Parametersammlung nach Vermessung der Reinsubstanzen hinzugefügt werden.

Vorzugsweise erfolgt die Bestimmung der Molekülstruktur mittels NMR-Spektren, der Retentionszeit und der Molmasse der isolierten Reinsubstanzen und eine Verifizierung der in der Paramtersammlung enthaltenen Parameter. Nach der Auftrennung eines erfolgversprechenden Gemisches werden also die erhaltenen Reinsubstanzen mit dem gleichen standardisierten Verfahren charakterisiert, das auch zur Beurteilung der Gemische eingesetzt wird. Zusätzlich werden von jeder isolierten Reinsubstanz $^1$H-NMR-Spektren aufgenommen. Durch Vergleich der erhaltenen Parameter mit den in der Parametersammlung vorhandenen Parametern der bereits vermessenen Reinsubstanzen kann in vielen Fällen eine Zuordnung der Struktur erfolgen, die durch Vergleich der $^1$H-NMR-Spektren bestätigt werden kann.

Es hat sich gezeigt, dass die Molmasse zusammen mit den relativen Retentionszeiten eine ausreichende Charakterisierung der Strukturen insofern darstellen, als Redundanz vermieden werden soll und nur hinreichend vielversprechende Gemische getrennt werden sollen. Die Werte der Parametersammlung können mit den nach der Trennung in Reinsubstanzen ermittelten Parametern korrigiert werden. Dadurch werden Matrix- und Konzentrationseffekte des Gemisches berücksichtigt, die bei den verschiedenen Gemischen zu unterschiedlichen Werten für gleiche Verbindungen führen können.

In einer bevorzugten Ausgestaltung der Erfindung erfolgt die Ermittlung der Molekülstruktur von Verbindungen, die nicht in der internen Parametersammlung enthalten sind, durch

(aa) Datenbankrecherche bezüglich aller Strukturen, welche die Molmasse innerhalb eines vorgegebenen Toleranzbereichs aufweisen,

(bb) Bestimmung eines Strukturfragments aus dem NMR-Spektrum der Reinsubstanz,

(cc) Auswahl aller Strukturen der in (aa) ermittelten Strukturen, die dieses Strukturfragment aufweisen,

(dd) Wiederholen von (bb) und (cc) mit weiteren Strukturfragmenten, bis nur eine oder keine Struktur bleibt,

(ee) Auswahl aller Reinsubstanzen für die keine Datenbank eine Struktur liefert, und Strukturbestimmung durch Aufnahme und Interpretation weiterer NMR-Spektren.

Bei dieser Methode werden bekannte Strukturen sehr schnell erkannt und brauchen nicht mehr aufgeklärt werden. Der Durchsatz kann durch die Vermeidung von Redundanz an dieser Stelle weiter erhöht werden. Außerdem ist die Strukturaufklärung bis zu dem Punkt, an dem festgestellt wird, daß die Struktur nicht in Datenbanken vorliegt, erheblich einfacher, da nur Strukturfragmente erkannt werden müssen. Dadurch bietet sich die Möglichkeit der Automatisierung, die bei der normalen Strukturaufklärung normalerweise nicht gegeben ist, bzw. nur mit erheblichem Aufwand zu realisieren ist. Vorzugsweise werden solche Strukturen bei der Auswahl vernachlässigt, deren Strukturfragmente einen Widerspruch zu dem Spektrum der ausgewählten Reinsubstanz bewirken. Das bedeutet, daß in den durch die Datenbank angebotenen Strukturen nach Strukturfragmenten mit charakteristischen Signalmustern im $^1$H-NMR-Spektrum gesucht und eine Überprüfung vorgenommen wird, ob diese Signalmuster in dem an der Reinsubstanz gemessenen Spektrum vorliegen. Die Signalmuster werden entweder durch Simultionsprogramme aus der Struktur berechnet oder durch einen Bearbeiter mit der entsprechenden Erfahrung vorhergesagt. Die Anzahl der Strukturfragmente, die bei der Aufklärung bekannt sein müssen, kann bei geschickter Auswahl charakteristischer Fragmente weiter reduziert werden.

Die Molmasse wird durch Auswertung von Massenspektren ermittelt. Es hat sich überraschenderweise gezeigt, dass bei Verwendung von positiven und negativen Elektrospray-Ionisierungen die Molmassen der Peaks aus Gemischen automatisch mit befriedigender Richtigkeit zu berechnen sind. Es sind auch andere in der Massenspektrometrie übliche Ionisierungsmethoden einsetzbar, sofern die Möglichkeit eines Schaltens der Polaritäten besteht. Dem Laufmittel wird vorzugweise ein Puffer zugesetzt. Dabei hat sich Ammoniumformiat für die vorliegende Anwendung als besonders geeignet erwiesen. Es ist aber auch die Verwendung von Ammoniumacetat möglich.

Die Verwendung von organischen Puffersystemen in der mobilen Phase der HPLC-Chromatographie erhöht die Anzahl von spezifischen Addukten im Massenspektrum, so daß die Sicherheit der Bestimmung der Molmassen erhöht wird. Bei Verwendung von Ammoniumformiat als Puffersystem können insbesondere folgende Addukte auftreten:

Im positiven Massenspektrum können als mutmaßliche Addukte [M+H]⁺, [M+NH4]⁺ und [M+Na]⁺ vorliegen, die durch Subtraktion von 1, 18 bzw 23 vom gemessenen M7z-Wert zu jeweils der gleichen Molmasse führen. Im negativen Massenspektrum können als mutmaßliche Addukte [M-H]⁻ und [M+HCOO]⁻ vorliegen, die durch Addition von 1 bzw. Subtraktion von 45 vom gemessenen m/z-Wert zu jeweils der gleichen Molmasse führen.

In einer bevorzugten Ausgestaltung der Erfindung werden jeweils die 5 intensivsten m/z-Werte des mit positiver und des mit negativer Ionisierung aufgenommenen Massenspektrums zur Berechnung der Molmassen herangezogen. Üblicherweise treten nicht alle Addukte zu jeder Molmasse auf, das gleichzeitige Auftreten von zwei oder mehr Addukten ist aber im allgemeinen ein ausreichender Beweis für die vorliegende Molmasse. Wenn mehrere Molmassen in einem Massenspektrum denkbar sind, muß geprüft werden, ob es sich um Di- oder Oligomere handelt, die sich auf ein Monomer zurückführen lassen. Di- und Oligomere treten unter den Bedingungen der Ionisierung im Massenspektrometer auf. Ebenfalls denkbar ist das Auftreten von Fragmenten, die durch Abspaltung von neutralen oder geladenen Teilchen aus dem Muttermolekül entstehen. Diese charakteristischen Fragmente können ebenfalls in die Parametersammlung aufgenommen und zur Identifizierung herangezogen werden. In den meisten Fällen stellt die größte ermittelte Molmasse, bei der es sich nicht um Di- oder Oligomere handelt, die korrekte Molmasse dar.

Einer der physikalischen Parameter, die am nicht getrennten Gemisch gemessen werden, kann die Retentionszeit der Peaks bei der HPLC-Trennung sein.

Vorzugsweise wird die Quantifizierung und/oder Bestimmung der Retentionszeit mittels eines an eine HPLC-Säule gekoppelten Lichtstreudetektors (ELSD) vorgenommen. Der Lichtstreudetektor wird eingesetzt, da die Integrale der weitgehend strukturunabhängigen Signale besonders zur Quantifizierung geeignet sind.

Die Retentionszeiten sind substanzspezifische Parameter, sofern immer die gleichen HPLC-Bedingungen verwendet werden. Dazu zählen insbesondere die stationäre und die mobile Phase, der Gradient, die Temperatur und die Flußrate.

Die Retentionszeit wird auf besonders vorteilhafte Weise relativ zu den Signalen einer Standardlösung bestimmt, die der Probe zugesetzt wurde. Das entspricht einem internen Standard.

Alternativ wird die Retentionszeit relativ zu den Signalen einer Standardlösung bestimmt, die eine oder mehrere Substanzen enthält, deren Signale zwischen den Messungen der Gemische aufgenommen werden. Das entspricht einem externen Standard. Dadurch wird erreicht, dass die Varianz der Retentionszeiten bei Verwendung geringfügig unterschiedlicher HPLC-Systeme minimiert wird. Die Varianz tritt z. B. auf bei unterschiedlichen HPLC-Anlagentypen mit unterschiedlichem Design von Mischkammern zur Generierung des Laufmittelgradienten oder mit unterschiedlichen Kapillarwegen, unterschiedliche Chargen der gleichen stationären Phase u.s.w..

Die absoluten Retentionszeiten werden auf relative Retentionszeiten umgerechnet. Die relativen Retentionszeiten werden dabei auf ein gemeinsames Standardsystem bezogen. Es wird also entweder jeder HPLC-Probe, die das Gemisch oder eine Reinsubstanz sein kann, ein Standard zugesetzt und die Retentionszeit jeder Fraktion relativ zu dem Signal des Standards bestimmt, oder die Retentionszeit wird relativ zu dem Signal eines Standards bestimmt, dessen Signal zwischen den Messungen an den Gemischen bestimmt wird.

Statt einer einzelnen Standardsubstanz kann auch ein Gemisch aus mehreren Verbindungen als Standard eingesetzt werden.

In einer Ausgestaltung der Erfindung wird jeweils ein Signal des Standards vor und nach einer oder mehreren Messungen der Gemische aufgenommen und die Retentionszeiten der Fraktionen eines Gemisches werden relativ zu einem interpolierten Wert für den Standard ermittelt. Die aktuellen Retentionszeiten der Standardsubstanzen können bei der Verwendung externer Standards in regelmäßigen Abständen, z. B. nach jedem zehnten HPLC-Lauf oder alle 8 Stunden ermittelt werden. Als Bezugspunkt der Berechnung werden die Retentionszeiten des Standardgemisches herangezogen, dessen HPLC-Lauf dem der Probe zeitlich am nächsten lag. Zur Berechnung der relativen Retentionszeiten wird zwischen den Retentionszeiten der Referenzsubstanzen, die der Retentionszeit der Probe am nächsten liegen, linear interpoliert. Es können aber auch andere Funktionen zur Interpolation herangezogen werden. Die lineare Interpolation kann mit Hilfe der Formel

$$\text{Rt\_calc} = \text{Rtref2} - [(\text{Rtref2} - \text{Rtref1}) * (\text{RtX2} - \text{Rt}) / (\text{RtX2} - \text{RtX1})]$$

erfolgen.
Dabei bezeichnen

Rt_calc :           relative Retentionszeit
Rt :                experimentell ermittelte Retentionszeit
Rtref1, Rtref2:     Retentionszeiten der ersten Referenzmessung
RtX1, RtX2 :        aktuelle Retentionszeiten des Standardgemisches

Die Signale des Standards und jeder Fraktion werden zur Quantifizierung auf einen vorgegebenen Lösungsmittelanteil normiert.

Die Quantifizierung kann durch Vergleich der Fläche der Peaks im Chromatogramm des Extraktes mit der Fläche eines Peaks aus dem Standardgemisch erfolgen. In erster Näherung kann davon ausgegangen werden, dass alle Substanzen bei gleicher Konzentration die gleiche Signalresponse im ELSD-Detektor aufweisen. Es ist aber auch möglich, z. B. durch Detektion per UV-Detektor zu ermitteln, um welche Substanzklasse es sich handelt und einen für diese Substanzklasse spezifischen Korrekturfaktor zu berücksichtigen.

Die gradientenabhängige Response des Signals im ELSD-Detektor kann folgendermaßen exerimentell ermittelt und rechnerisch berücksichtigt werden: Eine beliebige Substanz wird in jeweils gleicher Konzentration in ein mit einem ELSD-Detektor ausgestattetes HPLC-System ohne Trennsäule bei konstantem Fluß injiziert und die Fläche des Signals bei unterschiedlichen Zusammensetzungen der stationären Phase ermittelt. Die Auftragung von Signalfläche gegen die Laufmittelzusammensetzung liefert eine Funktion, die zur Korrektur der Flächen in Abhängigkeit der Laufmittelzusammensetzung eingesetzt werden kann.

Das Verfahren eignet sich besonders für den Aufbau von Substanzbibliotheken, deren Substanzen Extrakten aus Biomaterialien entstammen. Solche Biomaterialien können Pflanzen, Tiere, Pilze, Bakterien oder Teile davon sein.

Weitere Ausgestaltungen der Erfindung sind Gegenstand der Unteransprüche. Ein Ausführungsbeispiel ist nachstehend unter Bezugnahme auf die beigefügten Zeichnungen näher erläutert.

## Kurze Beschreibung der Zeichnungen

Fig. 1    ist eine schematische Darstellung des Prinzips zur Berechnung der relativen Retentionszeiten.

Fig 2    ist das HPLC-Chromatogramm eines Pflanzenextraktes, das mit ELSD (oben) und UV bei 254 nm (unten) aufgenommen wurde.

Fig 3    ist das HPLC-Chromatogramm eines Standardgemisches, das mit ELSD aufgenommen wurde.

Fig 4    zeigt das positive Massenspektrum des Peaks bei Rt = 17,77 min in Fig. 3

Fig. 5    zeigt das zu Fig.4 zugehörige negative Massenspektrum

## Beschreibung der Ausführungsbeispiele

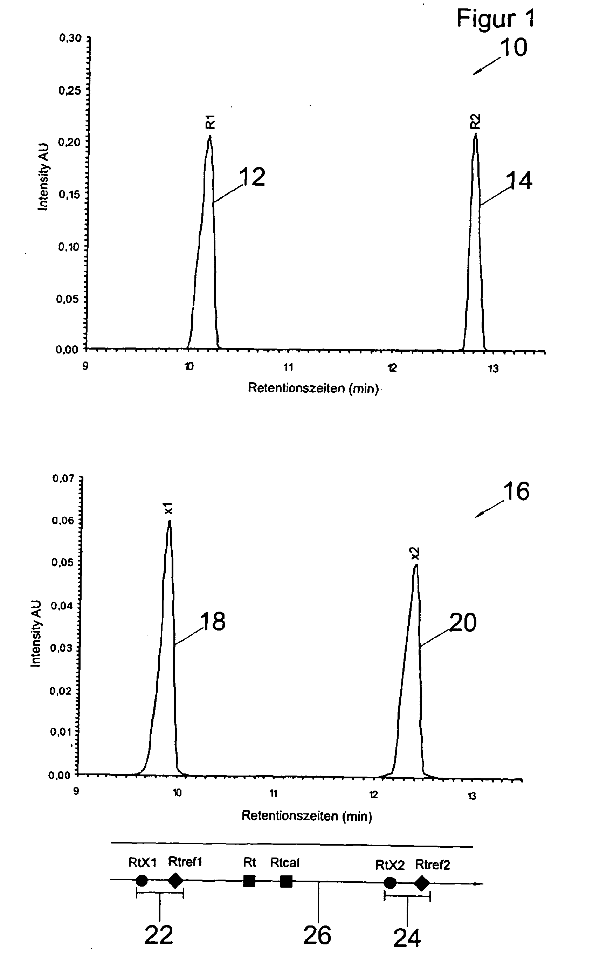

In Fig. 1 ist das Prinzip der Berechnung der relativen Retentionszeiten schematisch dargestellt. Das HPLC-Chromatogramm 10 einer Standardlösung unter Standardbedingungen weist zwei Peaks 12 und 14 bei Retentionszeiten von Rtref1=10,15 min und Rtref2=12,85 min auf. In einem später unter Laborbedingungen aufgenommenen HPLC-Chromatogramm 16 treten die korrespondierenden Peaks 18 und 20 der gleichen Substanzen bei RtX1=9,85 min und RtX2=12,30 min auf. Die Peaks 18 und 20 sind also um Distanzen 22 bzw. 24 gegenüber den Peaks 12 und 14 verschoben. Dies ist auf der Zeitachse 26 nocheinmal dargestellt. Die relative Retentionszeit Rt_calc einer Substanz in einem Gemisch, die einen Peak bei Rt=11,40 min unter den gleichen Laborbedingungen aufweist, ist dann Rt_calc = Rtref2-[(Rtref2-Rtrefl)*(RtX2-Rt)/(RtX2-RtX1)]= 11,86 min. Die Verschiebung des Substanzpeaks durch veränderte Bedingungen gegenüber den Standardbedingungen beträgt also -0,46 min. In dem folgenden Beispiel werden die relativen Retentionszeiten ebenfalls auf diese Weise mit einem externen Standard berechnet.

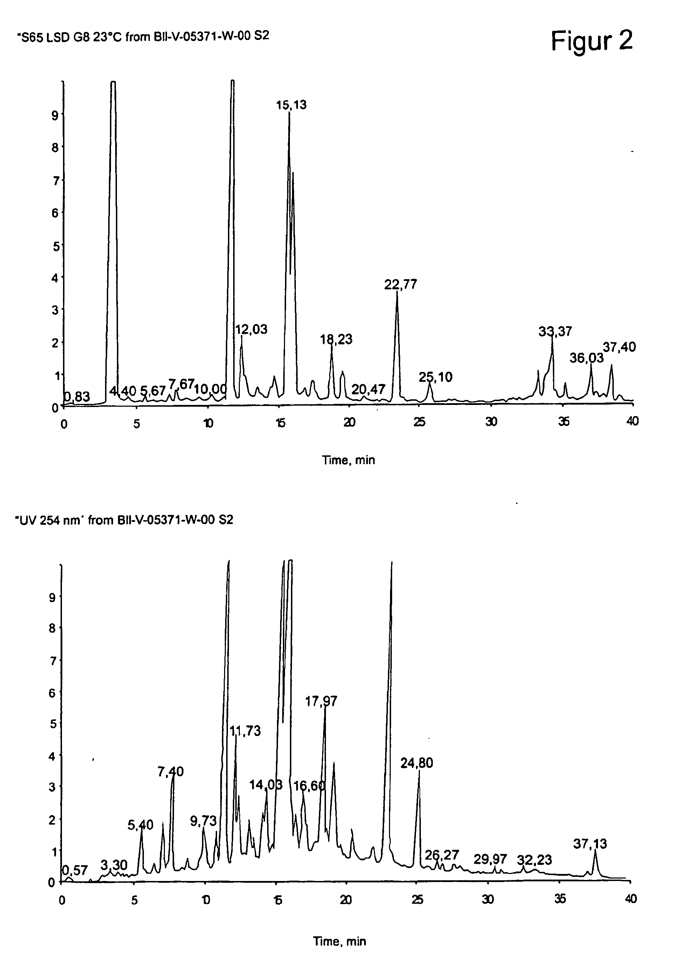

Ein Pflanzenextrakt wird in einer Konzentration von 10mg/ml Methanol gelöst und per HPLC/MS/ELSD vermessen. Fig. 2 zeigt das Chromatogramm (ELSD-Detektion) des Extraktes. Außerdem ist ein - zeitlich leicht verschobenes - Chromatogramm dargestellt, das mittels UV bei 254 nm aufgenommen wurde.

Unter gleichen HPLC-Bedingungen wird direkt nach Vermessen des Extraktes an der gleichen HPLC-Anlage ein Gemisch aus folgenden Referenzsubstanzen vermessen: D-Lactose (2,09), Gallussäure (4,09), Catechin (7,69), Boldine (10,01) Umbelliferon (12,63), Ajmalicin (16,39), Steviosid (18,12), Oleandomycindiacetat (20,79), Cholsäure (23,28), Lovastatin (26,45), Abietinsäure (29,79) und Parigenin (31,68). Die hinter der Bezeichnung in Klammern angegebenen Werte bezeichnen die Vergleichs-Retentionszeiten.

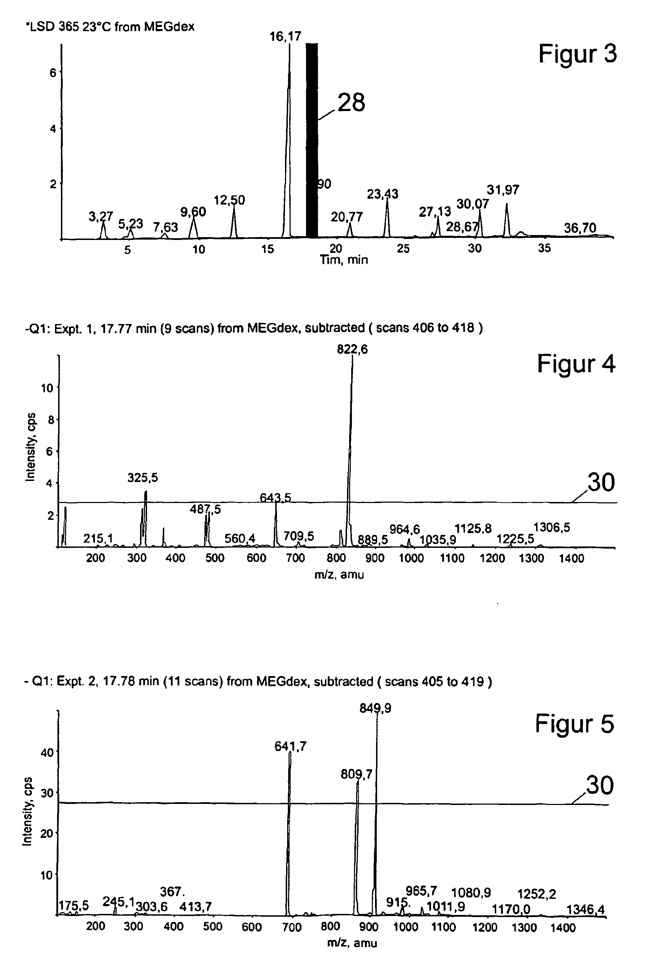

In Fig.3 ist das Chromatogramm dieser Refenzsubstanzmischung gezeigt. Das Chromatogramm wurde wiederum unter Laborbedingungen aufgenommen. Die Peaks sind leicht gegenüber den Referenzwerten verschoben. So ist der Peak von Umbelliferon nicht bei 12,63 sondern bei 12,50 min zu finden. Aus den beiden Chromatogrammen werden nach oben beschriebenem Verfahren die relativen Retentionszeiten berechnet.

Für jeden Peak des Gemisches, der eine bestimmte minimale Fläche übersteigt, werden die Retentionszeit, die absolute Fläche des Peaks, das Datum und die Uhrzeit der Injektion der Probe und die 5 intensivsten m/z-Werte der (+)- und (-)-ESI-Spektren in eine Access-Datenbank importiert. Zur Berechnung der zu erwartenden Ausbeuten der einzelnen Substanzen wird ein Peak des externen Standards (hier: Umbelliferon) herangezogen und die Masse des insgesamt zugänglichen Biomaterials berücksichtigt. Die Gradientenabhängigkeit der Signalresponz des ELSD-Detektors wird über eine empirisch ermittelte Funktion berücksichtigt und die experimentellen Peakflächen entsprechend korrigiert. Die Ergebnisse der Berechnung der relativen Retentionszeiten und erwarteten Ausbeuten sind in der folgenden Tabelle aufgeführt. In dieser Tabelle sind die relevanten Peaks nach Auswertung des ELSD-Chromatogramms zur Quantifizierung gezeigt.

| Probenname | Rt_exp | Rt_rel | Area | Prognose (mg) |
|---|---|---|---|---|
| V-05371-W-00 S2 | 7,67 | 7,65 | 48,44 | 140,28 |
| V-05371-W-00 S2 | 9,13 | 9,35 | 48,29 | 132,77 |
| V-05371-W-00 S2 | 10,00 | 10,29 | 43,56 | 116,51 |
| V-05371-W-00 S2 | 11,17 | 11,37 | 1703,42 | 4417,87 |
| V-05371-W-00 S2 | 12,03 | 12,17 | 180,55 | 458,04 |
| V-05371-W-00 S2 | 14,30 | 14,49 | 141,68 | 337,87 |
| V-05371-W-00 S2 | 15,13 | 15,37 | 1134,78 | 2646,54 |
| V-05371-W-00 S2 | 15,43 | 15,68 | 1207 | 2792,73 |
| V-05371-W-00 S2 | 16,37 | 16,65 | 83,91 | 189,57 |
| V-05371-W-00 S2 | 16,90 | 17,16 | 133,08 | 296,97 |
| V-05371-W-00 S2 | 18,23 | 18,44 | 238,69 | 516,70 |
| V-05371-W-00 S2 | 19,03 | 19,21 | 150,3 | 319,59 |
| V-05371-W-00 S2 | 22,77 | 22,73 | 477,92 | 940,22 |
| V-05371-W-00 S2 | 25,10 | 24,79 | 80,3 | 151,33 |

Die Ermittlung der Molmasse sei exemplarisch am Peak 28 in Fig.3 von Steviosid bei einer Retentionszeit von 17.9 min aus dem Standardgemisch erläutert. Fig. 4 und Fig.5 zeigen die zugehörigen Massenspektren. In Fig. 4 ist das positive und in Fig. 5 das negative Massenspektrum dargestellt. Für jeden exportierten m/z-Wert, der eine bestimmte relative Intensität, dargestellt durch den Schwellwert 30 übersteigt, werden alle denkbaren Molmassen berechnet. Dabei werden die möglicherweise auftretenden Addukte berücksichtigt. Der Peak bei m/z = 822,6 könnte zum Beispiel: auf die Molmasse MW = 821 (822 =[M+H]$^+$) oder auf MW = 804 (822 = [M+NH$_4$]$^+$) oder auf MW = 799 (822 = [M+Na]$^+$) zurückzuführen sein.

Das Ergebnis der Berechnung ist in folgender Tabelle zusammengefasst:

| | M/z | +H | +NH$_4$ | +Na | -H | +HCOO$^-$ |
|---|---|---|---|---|---|---|
| (+)-ESI | 822,6 | 821 | | 799 | | |
| | 643,5 | 642 | 625 | 620 | | |
| (-)-ESI | 849,9 | | | | 850 | |
| | 803,7 | | | | | 758 |
| | 641,7 | | | | 642 | 596 |

Im nächsten Schritt werden die auftretenden Massen gezählt und entsprechend der Häufigkeit ihres Auftretens mit Punkten bewertet. Alle Molmassen, die mindestens zwei Mal auftreten, werden in die Berechnung von Fragmenten und Addukten einbezogen.

Die Bewertung und Priorisierung der denkbaren Molmassen mit mindestens zwei Punkten ohne die Berücksichtigung von Fragmenten ergibt drei Punkte für die Molmasse 804 und zwei Punkte für die Molmasse 642. Weitere Molmassen mit mindestens zwei Punkten wurden nicht ermittelt.

Wenn die untersuchten Substanzen glykosyliert oder verestert sind, ist unter ESI-Bedingungen im Massenspektrometer häufig die Abspaltung der Zucker bzw. Ester zu beobachten. Da bestimmte Zucker und Ester in niedermolekularen Naturstoffen häufig auftreten, können diese Fragmente bei der Auswertung der Massenspektren berücksichtigt werden. Wenn die Differenz zwischen zwei ermittelten Molmassen der Differenz eines häufig auftretenden Fragmentes entspricht, ist das Auftreten ein weiterer Beweis für die Molmasse der Muttersubstanz. Im vorliegenden Beispiel beträgt die Differenz der beiden ermittelten Molmassen 162 Einheiten, eine Differenz, die auf des Vorliegen des Strukturelementes einer Hexose (z.B. Glucose) hindeutet.

Die Punktzahl der Molmasse des Fragmentes wird zu der Punktzahl der Molmasse der Muttersubstanz addiert. Die Molmasse, die am meisten Punkte aufweist, wird als korrekte zu dem Peak gehörige Molmasse angesehen. Unter Berücksichtigung der Fragmente ergibt sich in diesem Beispiel eine Bewertung mit der Punktzahl fünf für die Molmasse 804 und eine Punktzahl 2 für die Molmasse 642. Die Substanz mit der Molmasse 804 enthält demnach (vermutlich) Hexose, während die Substanz mit der Molmasse 642 der Substanz mit der Molmasse 804 entspricht, bei der eine Abspaltung einer Hexose vorlag. Die voll automatisch ermittelte Nominalmasse beträgt also 804 g/Mol.

Die Peaks im vorliegenden Beispiel sind durch die in folgender Tabelle zusammengefaßten Parameter charakterisiert.

| # | Rt_exp | Rt_rel | MW | Enthaltene Strukturelemente |
|---|---|---|---|---|
| 1 | 7,67 | 7,65 | 287 | |
| 2 | 9,13 | 9,35 | 598 | |
| 3 | 10,00 | 10,29 | 426 | |
| 3 | 10,00 | 10,29 | 540 | |
| 4 | 11,17 | 1,37 | 378 | Hexose |
| 5 | 12,03 | 12,17 | 740 | |
| 6 | 14,30 | 14,49 | 216 | |
| 6 | 14,30 | 14,49 | 769 | |
| 7 | 15,13 | 15,37 | 378 | Hexose |
| 8 | 15,43 | 15,68 | 0 | |
| 9 | 16,37 | 16,65 | 464 | |
| 10 | 16,90 | 17,16 | 190 | |
| 10 | 16,90 | 17,16 | 270 | |
| 11 | 18,23 | 18,44 | 524 | |
| 12 | 19,03 | 19,21 | 286 | |
| 12 | 19,03 | 19,21 | 316 | |
| 13 | 22,77 | 22,73 | 300 | |
| 13 | 22,77 | 22,73 | 330 | |
| 14 | 25,10 | 24,79 | 270 | |

Die Molmasse Null (0) wird eingetragen, wenn nach dem oben erläuterten Algorithmus keine Molmasse aus den Rohdaten ermittelt werden kann. Die Zusammenfassung der Parameter der strukturellen Bewertung der relevanten Peaks dient als Basis für die Suche in Datenbanken. Die Suche in einer internen Datenbank, die die relativen Retentionszeiten, Molmassen und Strukturen von Reinsubstanzen enthält, führt zu dem Ergebnis, dass eine Verbindung, deren Daten denen von Peak 2 im Rahmen akzeptabler Fehlergrenzen (Nominalmasse der Verbindung entspricht der ermittelten Molmasse des Peaks und die relative Retentionszeit weicht um nicht mehr als +/- 0,15 min von der des Peaks ab) entsprechen, einmal gefunden werden konnte. Es handelt sich bei Peak 2 um eine mutmaßlich redundante Verbindung. Wenn der Algorithmus zur Ermittlung der Molmassen zwei Molmassen mit gleicher Punktzahl liefert, werden beide Massen unabhängig zur Suche in der internen Datenbank verwendet. Es wird postuliert, dass es sich um zwei coeluierende Verbindungen handelt.

Im nächsten Schritt wird ermittelt, wie häufig Peaks mit im Rahmen der oben erwähnten Fehlergrenzen gleichen Parametersätzen in einem bestimmten Extraktkontingent auftreten. Jeder Peak im Extrakt wird mit einer Punktzahl bewertet, die dem Kehrwert der Häufigkeit entspricht. Die Gesamtpunktzahl des Extraktes ergibt sich als Summe der Punktzahlen der einzelnen Peaks. Auf diese Weise läßt sich eine Rangfolge der potentiellen Ergiebigkeit der Extrakte erstellen.

**Patentansprüche**

1.  Verfahren zum Auffinden von Stoffgemischen, die zum Aufbau von Substanzbibliotheken geeignet sind, welche niedermolekulare, organische Substanzen enthalten, mit den Schritten

    (a) Aufbereitung der Gemische für Analysezwecke
    (b) Trennung der Gemische mittels chromatographischer oder elektrophoretischer Methoden, und
    (c) Identifizierung von niedermolekularen, organischen Substanzen in dem jeweiligen Gemisch durch Erstellung eines Profils aus wenigstens zwei physikalischen Parametern für jedes Gemisch durch Detektion jeder Fraktion mit wenigstens zwei geeigneten Detektoren zur Charakterisierung der in dem Gemisch enthaltenen Substanzen, Vergleich der so erhaltenen Parameter mit den Parametern einer Parametersammlung bekannter Substanzen und Ermittlung der in dieser Sammlung bekannten Substanzen für jedes Gemisch,
    **dadurch gekennzeichnet, daß**
    (d) eine Quantifizierung der so identifizierten neuen und bekannten Substanzen vorgenommen wird,
    (e) die Gemische, bei welchen ausgewählte Substanzen in einer Mindestzahl und/oder Mindestmenge vorliegen, ausgewählt werden,
    (f) der erste physikalische Parameter die Retentionszeit ist und
    (g) der zweite physikalische Parameter die Molmasse ist, die mittels Massenspektrometrie ermittelt wird.

2.  Verfahren nach Anspruch 1, **dadurch gekennzeichnet, daß** aus den in Schritt (e) ausgewählten Gemischen Reinsubstanzen isoliert und einer Substanzbibliothek hinzugefügt werden.

3.  Verfahren nach Anspruch 1 oder 2, **dadurch gekennzeichnet, daß** die Substanzbibliothek nur solche Substanzen umfaßt, deren Parameter in der Parametersammlung vorhanden sind.

4.  Verfahren nach Anspruch 2 oder 3, **gekennzeichnet durch** die Bestimmung der Molekülstruktur mittels NMR-Spektren, der Retentionszeit und Molmasse der isolierten Reinsubstanzen und Verifizierung der in der Parametersammlung enthaltenen Parameter.

5.  Verfahren nach Anspruch 4, **dadurch gekennzeichnet, daß** die Ermittlung der Molekülstruktur erfolgt durch

    (aa) Datenbankrecherche bezüglich aller Strukturen, welche die Molmasse innerhalb eines vorgegebenen Toleranzbereichs aufweisen,
    (bb) Bestimmung eines Strukturfragments aus dem NMR-Spektrum der Reinsubstanz,
    (cc) Auswahl aller Strukturen der in (aa) ermittelten Strukturen, die dieses Strukturfragment aufweisen,
    (dd) Wiederholen von (bb) und (cc) mit weiteren Strukturfragmenten, bis nur eine oder keine Struktur bleibt,
    (ee) Auswahl aller Reinsubstanzen für die keine Datenbank eine Struktur liefert, und Strukturbestimmung durch Aufnahme und Interpretation weiterer NMR-Spektren.

6.  Verfahren nach einem der vorgehenden Ansprüche, **dadurch gekennzeichnet, daß** die Molmasse über das Massenspektrum mittels eines Massenspektrometers ermittelt wird, welches mittels einer Elektrospray-Ionisierung an eine HPLC-Säule gekoppelt ist.

7.  Verfahren nach Anspruch 6, **dadurch gekennzeichnet, daß** sowohl positive als auch negative Ionisierungsmethoden der Massenspektrometrie eingesetzt werden.

8.  Verfahren nach einem der Ansprüche 6 oder 7, **dadurch gekennzeichnet, daß** dem Laufmittel ein Puffer zugesetzt wird.

9.  Verfahren nach Anspruch 8, **dadurch gekennzeichnet, daß** der Puffer Ammoniumformiat ist.

10. Verfahren nach Anspruch 9, **dadurch gekennzeichnet, daß** bei der Auswertung des positiven Massenspektrums Peaks mit einer gegenüber der zu ermittelnden Molmasse um 1, 18 und/oder 23 erhöhten Massenzahl berücksichtigt werden.

11. Verfahren nach Anspruch 9 oder 10, **dadurch gekennzeichnet, daß** bei der Auswertung des negativen Massenspektrums Peaks mit einer gegenüber der zu ermittelnden Molmasse um 45 erhöhten oder um 1 verringerten Massenzahl berücksichtigt werden.

12. Verfahren nach einem der vorgehenden Ansprüche, **dadurch gekennzeichnet, daß** einer der physikalischen Parameter, die in Schritt (c) am nicht-getrennten Extrakt gemessen werden, die Retentionszeit der Peaks bei der HPLC-Trennung ist.

13. Verfahren nach einem der vorgehenden Ansprüche, **dadurch gekennzeichnet, daß** die Quantifizierung und/oder die Bestimmung der Retentionszeit mittels eines an eine HPLC-Säule gekoppelten Lichtstreudetektors (ELSD) vorgenommen wird.

14. Verfahren nach Anspruch 13, **dadurch gekennzeichnet, daß** die Retentionszeit relativ zu dem Signalen einer Standardlösung bestimmt wird, die der Probe zugesetzt wurde.

15. Verfahren nach Anspruch 13, **dadurch gekennzeichnet, daß** die Retentionszeit relativ zu den Signalen einer Standardlösung bestimmt wird, die eine oder mehrere Substanzen enthält, deren Signale zwischen den Messungen der Gemische aufgenommen werden.

16. Verfahren nach Anspruch 15, **dadurch gekennzeichnet, daß** jeweils ein Signal des Standards vor und nach einer oder mehreren Messungen der Gemische aufgenommen wird und die Retentionszeiten der Fraktionen eines Gemischs relativ zu einem interpolierten Wert für den Standard ermittelt werden.

17. Verfahren nach einem der Ansprüche 14 bis 16, **dadurch gekennzeichnet, daß** die Signale des Standards und jeder Fraktion auf einen vorgegebenen Lösungsmittelanteil normiert werden.

18. Verfahren nach einem der Ansprüche 4 bis 17, **dadurch gekennzeichnet, daß** die Werte der Parametersammlung mit den nach der Trennung in Reinsubstanzen ermittelten Parametern korrigiert werden.

19. Verfahren nach einem der vorgehenden Ansprüche, **dadurch gekennzeichnet, daß** die Parametersammlung als zusätzliche Parameter den Namen und die Herkunft der Substanz umfasst.

20. Verfahren nach einem der vorgehenden Ansprüche, **dadurch gekennzeichnet, daß** die Gemische Extrakte aus Biomaterialien sind.

21. Vorrichtung zur Durchführung des Verfahrens nach einem der Ansprüche 1 bis 20, enthaltend

   (a) eine HPLC-Säule,
   (b) ein mit Elektrospray-Ionisierung an die HPLC-Säule gekoppeltes Massenspektrometer,
   (c) Rechnermittel zur Bestimmung der Molekülmasse aus den Massenspektren,
   (d) einen an die HPLC-Säule gekoppelten Lichtstreu-Detektor,
   (e) Speichermittel zum Speichern und Abrufen der Retentionszeit und der Molekülmasse in einer Parametersammlung
   (f) Trennmittel zum Trennen der in dem Extrakt enthaltenen Reinsubstanzen, und
   (g) Rechnermittel zur Bestimmung der Molekülstruktur.

22. Vorrichtung nach Anspruch 21, **dadurch gekennzeichnet, daß** eine weitere HPLC-Säule vorgesehen ist, die alternativ zu der ersten Säule über ein Ventil einkoppelbar ist.

23. Vorrichtung nach einem der Ansprüche 21 und 22, **dadurch gekennzeichnet, daß** an dem Massenspektrometer Mittel zum Umstellen von einem Ionisationsmodus in den entgegengesetzten Ionisationsmodus vorgesehen sind.

24. Verwendung eines Verfahrens nach einem der Ansprüche 1 bis 20 zur Identifizierung von Reinsubstanzen in Extrakten.

25. Verwendung eines Verfahrens nach einem der Ansprüche 1 bis 20 zum Vergleich von Extrakten bezüglich ihrer Ergiebigkeit hinsichtlich der darin vorliegenden Substanzen.

26. Verwendung eines Verfahrens nach einem der Ansprüche 1 bis 20 zur Identifizierung von synthetischen Verbindungen aus Reaktionsansätzen.

**Claims**

1. Method for finding substance mixtures suitable for the collection of a substance library comprising low molecular organic substances with the steps:

(a) preparing the mixtures for the purpose of analysing;
(b) separating the mixtures by means of chromatographic or electrophoretic methods; and
(c) identifying low molecular organic substances in the respective mixture by generating a profile of at least two physical parameters for each mixture by detection of each fraction with at least two suitable detectors for the characterization of the substances comprised in the mixture, comparing the parameters obtained in such way with the parameters in a parameter collection of known substances and determining for each mixture the known substances comprised in this collection;
**characterized in that**
(d) the new and known substances identified in such way are quantified,
(e) the mixtures are selected where selected substances are present in a minimum number and/or minimum quantity;
(f) the first physical parameter is the retention time and
(g) the second physical parameter is the molar mass determined by mass spectrometry.

2. Method according to claim 1, **characterized in that** pure substances are isolated from the mixtures selected in step (e) and added to a substance library.

3. Method according to claim 1 or 2, **characterized in that** the substance library comprises only substances with parameters present in the parameter collection.

4. Method according to claim 2 or 3, **characterized by** the determination of the molecular structure by means of NMR-spectra, the retention time or the molar mass of the isolated pure substance and verification of the parameters comprised in the parameter collection.

5. Method according to claim 4, **characterized in that** the determination of the molecular structure is achieved by

(aa) data base search with respect to all structures having the molar mass within a given tolerance range,
(bb) determination of a structure fragment from the NMR-spectrum of the pure substance,
(cc) selection of all structures of the structures determined in (aa) having this structure fragment,
(dd) repeating (bb) and (cc) with further structure fragments until only one or no structure remains,
(ee) selection of all pure substances for which the data base does not provide a structure and determining the structure by obtaining and interpreting further NMR-spectra.

6. Method according to any of the foregoing claims, **characterized in that** the molar mass is determined by the mass spectrum with a mass spectrometer which is coupled to a HPLC-column by means of a electrospray ionisation.

7. Method according to claim 6, **characterized in that** positive as well as negative ionisation methods of mass spectrometry are used.

8. Method according to claim 6 or 7, **characterized in that** a buffer is added to the mobile phase.

9. Method according to claim 8, **characterized in that** the buffer is ammonium formiate.

10. Method according to claim 9, **characterized in that** during the evaluation of the positive mass spectrum peaks with a molar mass increased by the mass number of 1, 18 and/or 23 with respect to the molar mass to be determined are considered.

11. Method according to claim 9 or 10, **characterized in that** during the evaluation of the negative mass spectrum peaks with a molar mass increased by a mass number of 45 or decreased by 1 with respect to the molar mass to be determined are considered.

12. Method according to any of the foregoing claims, **characterized in that** one of the physical parameters measured in step (c) of the not-separated extract is the retention time of the peaks of a HPLC-separation.

13. Method according to any of the foregoing claims, **characterized in that** the quantification and/or the determination of the retention time is carried out by means of a light scattering detector (ELSD) coupled to the HPLC-column.

14. Method according to claim 13, **characterized in that** the retention time is determined relative to the signals of a standard solution added to the sample.

15. Method according to claim 13, **characterized in that** the retention time is determined relative to the signals of a standard solution comprising one or more substances where the signals have been measured between the measurements of the mixtures.

16. Method according to claim 15, **characterized in that** one signal of the standard is recorded before and after one or more measurements of the mixtures, respectively, and the retention times of the fractions of the mixtures are determined relative to the interpolated value of the standard.

17. Method according to any of the claims 14 to 16, **characterized in that** the signals of the standards and of each fraction is normed to a given solvent portion.

18. Method according to any of the claims 4 to 17, **characterized in that** the values of the parameter collection are corrected with the parameters determined after the separation of the pure substances.

19. Method according to any of the foregoing claims, **characterized in that** the parameter collection comprises the name and the origin of the substance as an additional parameter.

20. Method according to any of the foregoing claims, **characterized in that** the mixtures are extracts of biological materials.

21. Device for carrying out the method according to any of the claims 1 to 20, comprising:

  (a) a HPLC-column,
  (b) a mass spectrometer coupled to the HPLC-column by electrospray ionisation,
  (c) computer means for determining the molecular mass from the mass spectra,
  (d) a light scattering detector coupled to the HPLC-column,
  (e) storage means for storing and providing the retention time and the molecular masses from a parameter collection
  (f) separating means for separating the pure substances comprised in the extract and
  (g) computer means for determining the molecular structure.

22. Device according to claim 21, **characterized in that** a further HPLC-column is provided adapted to be coupled in by a valve alternatively to the first column.

23. Device according to any of claims 21 and 22, **characterized in that** means are provided at the mass spectrometer for switching from one ionisation mode to the opposite ionisation mode.

24. Use of a method according to any of claims 1 to 20 for the identification of pure substances in extracts.

25. Use of a method according to any of claims 1 to 20 for comparing extracts relating to their yield with respect to the substances comprised therein.

26. Use of a method according to any of claims 1 to 20 for the identification of synthetic compounds from reaction preparations.

**Revendications**

1. Procédé de détection de mélanges de matières destiné à constituer des bibliothèques de substances comprenant des substances organiques de faible poids moléculaire, présentant les étapes de procédé de

(a) préparation des mélanges à des fins d'analyse,
(b) séparation des mélanges à l'aide de méthodes chromatographiques ou électrophorétiques, et
(c) identification de substances organiques de faible poids moléculaire contenues dans chaque mélange s'effectuant en établissant un profil d'au moins deux paramètres physiques pour chaque mélange intervenant par détection de chaque fraction à l'aide d'au moins deux détecteurs adéquats afin de caractériser les substances contenues dans le mélange, par comparaison des paramètres ainsi obtenus aux paramètres d'une collection de paramètres de substances connues, et par détermination des substances connues contenues dans ladite collection pour chaque mélange,
**caractérisé en ce que**
(d) une quantification des substances nouvelles et connues, ainsi identifiées, est réalisée,
(e) les mélanges dans lesquels des substances choisies sont présentes en nombre minimal et/ou en quantité minimale sont sélectionnés,
(f) le premier paramètre physique est le temps de rétention et
(g) le second paramètre physique est la masse molaire qui est déterminée par spectrométrie de masse.

2. Procédé selon la revendication 1, **caractérisé en ce que** des substances pures sont isolées à partir des mélanges sélectionnés à l'étape (e) et sont ajoutées à une bibliothèque de substances.

3. Procédé selon la revendication 1 ou 2, **caractérisé en ce que** la bibliothèque de substances ne comporte que les substances dont les paramètres sont présents dans la collection de paramètres.

4. Procédé selon la revendication 2 ou 3, **caractérisé par** la détermination de la structure moléculaire au moyen de spectres NMR, du temps de rétention et de la masse molaire des substances pures isolées et la vérification des paramètres contenus dans la collection de paramètres.

5. Procédé selon la revendication 4, **caractérisé en ce que** la détermination de la structure moléculaire s'effectue par

(aa) recherche dans une banque de données de toutes les structures qui présentent la masse molaire à l'intérieur d'une plage de tolérance prédéfinie,
(bb) détermination d'un fragment de structure à partir du spectre NMR de la substance pure,
(cc) sélection de toutes les structures des structures déterminées en (aa) qui présentent ledit fragment de structure,
(dd) réitération de (bb) et (cc) à l'aide d'autres fragments de structure jusqu'à ce qu'il ne reste plus qu'une structure ou plus aucune structure,
(ee) sélection de toutes les substances pures pour lesquelles aucune banque de données ne livre de structure, et détermination de la structure en acceptant et interprétant d'autres spectres NMR.

6. Procédé selon l'une quelconque des revendications précédentes, **caractérisé en ce que** la masse molaire est déterminée par l'intermédiaire du spectre de masse au moyen d'un spectromètre de masse qui est couplé à une colonne CLHP au moyen d'ionisation par électrospray.

7. Procédé selon la revendication 6, **caractérisé en ce qu'**on a recours autant aux méthodes de ionisation de spectrométrie de masse positive que négative.

8. Procédé selon l'une quelconque des revendications 6 ou 7, **caractérisé en ce qu'**on rajoute un tampon à la phase mobile.

9. Procédé selon la revendication 8, **caractérisé en ce que** le tampon est du formiate d'ammonium.

10. Procédé selon la revendication 9, **caractérisé en ce qu'**on prend en compte, lors de l'évaluation du spectre de masse positif, des pics présentant un nombre de masse supérieur à la masse molaire à déterminer de 1,18 et/ou 23.

11. Procédé selon la revendication 9 ou 10, **caractérisé en ce qu'**on prend en compte, lors de l'évaluation du spectre de masse négatif, des pics présentant un nombre de masse supérieur à la masse molaire à déterminer de 45 ou inférieur de 1.

12. Procédé selon l'une quelconque des revendications précédentes, **caractérisé en ce que** l'un des paramètres physiques qui sont mesurés à l'étape (c) sur l'extrait non séparé est le temps de rétention des pics lors de la séparation CLHP.

13. Procédé selon l'une quelconque des revendications précédentes, **caractérisé en ce que** la quantification et/ou la détermination du temps de rétention sont réalisées au moyen d'un détecteur évaporatif à diffusion de lumière (DEDL) couplé à une colonne CLHP.

14. Procédé selon la revendication 13, **caractérisé en ce que** le temps de rétention est déterminé par rapport aux signaux d'une solution étalon qui est rajoutée à l'échantillon.

15. Procédé selon la revendication 13, **caractérisé en ce que** le temps de rétention est déterminé par rapport aux signaux d'une solution étalon qui contient une ou plusieurs substances dont les signaux sont reçus entre les mesures des mélanges.

16. Procédé selon la revendication 15, **caractérisé en ce que** chaque signal de l'étalon est reçu avant et après une ou plusieurs mesures du mélange et les temps de rétention des fractions d'un mélange sont déterminés par rapport à une valeur interpolée de l'étalon.

17. Procédé selon l'une quelconque des revendications 14 à 16, **caractérisé en ce que** les signaux de l'étalon et chaque fraction sont normalisés jusqu'à obtention d'une part de solvant prédéfinie.

18. Procédé selon l'une quelconque des revendications 4 à 17, **caractérisé en ce que** les valeurs de la collection de paramètres sont corrigées à l'aide des paramètres déterminés après la séparation en substances pures.

19. Procédé selon l'une quelconque des revendications précédentes, **caractérisé en ce que** la collection de paramètres comprend sous forme de paramètres supplémentaires le nom et l'origine de la substance.

20. Procédé selon l'une quelconque des revendications précédentes, **caractérisé en ce que** les mélanges sont des extraits de biomatériaux.

21. Dispositif pour la réalisation du procédé selon l'une quelconque des revendications 1 à 20, comprenant,

> (a) une colonne CLHP,
> (b) un spectromètre de masse couplé à la colonne CLHP par ionisation par électrospray,
> (c) des moyens de calcul pour la détermination de la masse moléculaire à partir des spectres de masse,
> (d) un détecteur évaporatif à diffusion de lumière couplé à la colonne CLHP,
> (e) des moyens de mémorisation pour la mémorisation et l'appel du temps de rétention et de la masse moléculaire dans une collection de paramètres,
> (f) des moyens de séparation pour la séparation des substances pures contenues dans l'extrait, et
> (g) des moyens de calcul pour la détermination de la structure moléculaire.

22. Dispositif selon la revendication 21, **caractérisé en ce qu'**il est prévu une colonne CLHP supplémentaire qui peut être couplée en alternance à la première colonne par l'intermédiaire d'une soupape.

23. Dispositif selon l'une quelconque des revendications 21 et 22, **caractérisé en ce qu'**il est prévu sur le spectromètre de masse des moyens pour passer d'un mode d'ionisation dans le mode d'ionisation inverse.

24. Utilisation d'un procédé selon l'une quelconque des revendications 1 à 20 pour l'identification de substances pures dans des extraits.

25. Utilisation d'un procédé selon l'une quelconque des revendications 1 à 20 pour la comparaison d'extraits en ce qui concerne leur rendement par rapport aux substances qu'ils contiennent.

26. Utilisation d'un procédé selon l'une quelconque des revendications 1 à 20 pour l'identification de composés synthétiques à partir de préparations de réactions.

Figur 1

Figur 2

"LSD 365 23°C from MEGdex

Figur 3

16,17

28

90

3,27 5,23 7,63 9,60 12,50 20,77 23,43 27,13 30,07 31,97 28,67 36,70

5    10    15    20    25    30    35

Tim, min

-Q1: Expt. 1, 17.77 min (9 scans) from MEGdex, subtracted ( scans 406 to 418 )

822,6

Figur 4

Intensity, cps

30

325,5

487,5

643,5

215,1    560,4    709,5    889,5    964,6    1035,9    1125,8    1225,5    1306,5

200   300   400   500   600   700   800   900   1000   1100   1200   1300   1400

m/z, amu

- Q1: Expt. 2, 17.78 min (11 scans) from MEGdex, subtracted ( scans 405 to 419 )

849,9

Figur 5

641,7

30

809,7

Intensity, cps

367.

175,5    245,1 303,6    413,7    915.    965,7    1080,9    1252,2    1011,9    1170,0    1346,4

200   300   400   500   600   700   800   900   1000   1100   1200   1300   1400

m/z, amu